(19) Europäisches Patentamt
European Patent Office
Office européen des brevets

(11) Numéro de publication : **0 414 585 B1**

# (12) FASCICULE DE BREVET EUROPEEN

(45) Date de publication du fascicule du brevet : **21.04.93 Bulletin 93/16**

(51) Int. Cl.$^5$ : **A61K 7/13**

(21) Numéro de dépôt : **90402061.7**

(22) Date de dépôt : **18.07.90**

## (54) Composition de teinture des fibres kératiniques contenant un colorant indolique et au moins une paraphénylènediamine comportant un groupement amino secondaire et procédé.

(30) Priorité : **21.07.89 FR 8909835**

(43) Date de publication de la demande : **27.02.91 Bulletin 91/09**

(45) Mention de la délivrance du brevet : **21.04.93 Bulletin 93/16**

(84) Etats contractants désignés : **AT BE CH DE DK FR GB GR IT LI NL SE**

(56) Documents cités :
**GB-A- 2 205 329**
**GB-A- 2 207 443**

(73) Titulaire : **L'OREAL**
**14, Rue Royale**
**F-75008 Paris (FR)**

(72) Inventeur : **Grollier, Jean-François**
**16bis Boulevard Morland**
**F-75004 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 414 585 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à de nouvelles compositions de teinture des fibres kératiniques et plus particulièrement des fibres kératiniques humaines, telles que les cheveux, contenant un colorant indolique associé à au moins une paraphénylènediamine comportant un groupement amino secondaire ainsi que le procédé mettant en oeuvre ces compositions.

La couleur des cheveux, de la peau et des poils d'origine humaine provient principalement des pigments mélaniques secrétés par les mélanocytes. Ces pigments, d'origine naturelle, comprennent, en particulier, des pigments qu'on appelle des eumélanines.

Leur biosynthèse naturelle s'effectue en plusieurs étapes par polymérisation des produits d'oxydation d'un acide aminé : la tyrosine et l'un de ses produits d'oxydation est le 5,6-dihydroxyindole qui polymérise à son tour en eumélanine.

On a déjà proposé dans le passé de teindre les cheveux humains avec du 5,6-dihydroxyindole ou des colorants indoliques qui permettent de réaliser la teinture progressive des cheveux, c'est-à-dire d'obtenir une coloration présentant des nuances claires après une application du produit jusqu'à des teintes foncées par superposition des différentes applications.

La coloration s'effectue cependant lentement et pour accélérer la formation du pigment mélanique pour obtenir plus rapidement des nuances foncées, on a déjà décrit différents procédés, notamment dans les brevets français 1 133 594 et 1 166 172 mettant en oeuvre deux étapes et des agents oxydants, tels que du peroxyde d'hydrogène ou des agents d'oxydation catalytiques de nature métallique.

On a également préconisé d'autres agents d'oxydation tels que des ions iodure, des anions métalliques, des nitrites, des periodates, des sels de métaux rares.

La demanderesse vient de découvrir, ce qui fait l'objet de l'invention, qu'en utilisant une solution aqueuse contenant un colorant indolique et au moins une paraphénylènediamine monosubstituée sur l'un des groupements amino ou ses sels d'addition, il était possible d'obtenir très rapidement une teinture progressive des cheveux en des nuances naturelles allant du blond au noir.

Elle a constaté, en outre, qu'avec les compositions conformes à l'invention, le cheveu était teint plus rapidement dans des teintes foncées comparativement au procédé classique utilisant uniquement à titre de colorant le 5,6-dihydroxyindole.

Les compositions conformes à l'invention permettent une teinture progressive des cheveux humains à l'air ambiant et sans utilisation d'agent oxydant.

L'invention a donc pour objet des compositions tinctoriales pour fibres kératiniques, en particulier pour fibres kératiniques humaines, telles que les cheveux humains contenant à titre de colorant au moins un colorant indolique et au moins une paraphénylène diamine monosubstituée sur l'un des groupements amino.

Un autre objet de l'invention est constitué par le procédé de teinture mettant en oeuvre une telle composition.

D'autres objets de l'invention apparaîtront à la lecture de la description et des exemples qui suivent.

La composition tinctoriale conforme à l'invention est essentiellement caractérisée par le fait qu'elle contient dans un milieu aqueux approprié pour la teinture, un colorant indolique et au moins une paraphénylènediamine monosubstituée sur l'un des groupes amino ou ses sels d'addition d'acide.

Le colorant indolique correspond de préférence à la formule :

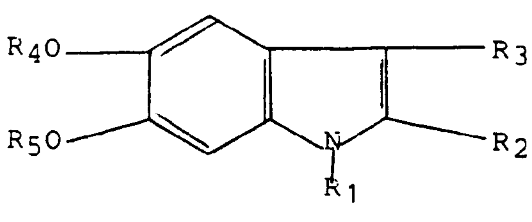

(I)

dans laquelle :

$R_1$ représente un atome d'hydrogène, un groupement alkyle inférieur ou un groupement $-SiR_6R_7R_8$;

$R_2$ et $R_3$, identiques ou différents, représentent un atome d'hydrogène ou bien un groupement alkyle inférieur, un groupement carboxyle, un groupement alcoxy, inférieur carbonyle ou un groupement $-COOSiR_6R_7R_6$;

$R_4$ et $R_6$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_6R_7R_6$, un groupement $-P(O)(OR_9)_2$, un groupement $SO_2OR_9$ ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :

$>P(O)OR_9$ ou bien $>CR_{10}R_{11}$;

$R_9$ et $R_{10}$ représentent un atome d'hydrogène ou un groupement alkyle inférieur, $R_{11}$ représentant un groupement alcoxy inférieur ou un groupement mono- ou dialkylamino, $R_6$, $R_7$ et $R_6$, identiques ou différents, représentant des groupements alkyle inférieurs, linéaires ou ramifiés;

et les sels d'addition avec des acides minéraux ou organiques ainsi que les sels de métaux alcalins, alcalino-terreux ou d'amines correspondants.

Les radicaux alkyle ou alcoxy inférieurs désignent de préférence des radicaux en $C_1$-$C_6$.

Parmi les composés préférés de formule (I), on peut citer plus particulièrement le 5,6-dihydroxyindole, le 2-méthyl 5,6-dihydroxyindole, le 5-méthoxy 6-hydroxy indole, le (5 ou 6)-acétoxy(6- ou 5-)hydroxyindole, le 2-carboxy, 5,6-dihydroxyindole, le 3-méthyl 5,6-dihydroxyindole, le 2,3-diméthyl 5,6-dihydroxyindole et leurs sels.

Le 5,6-dihydroxyindole est particulièrement préféré et il est utilisé tel quel ou sous l'une de ses formes protégées, comme par exemple le diacétate.

Les paraphénylènediamines monosubstituées sur l'un des groupements amino, utilisables conformément à l'invention, répondent à la formule :

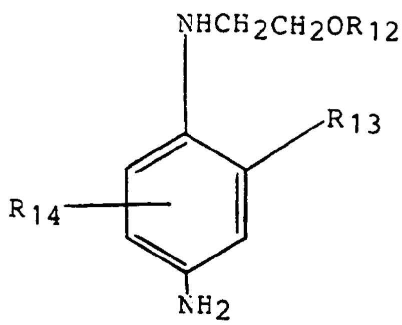

(II)

dans laquelle :

$R_{12}$ désigne un atome d'hydrogène ou un groupement alkyle inférieur;

$R_{13}$ désigne un atome d'hydrogène ou bien un groupement alkyle inférieur, alcoxy inférieur ou un atome d'halogène;

$R_{14}$ désigne un atome d'hydrogène, un groupement alkyle inférieur, alcoxy inférieur ou un atome d'halogène; $R_{14}$ occupant l'une quelconque des positions restantes du noyau benzénique.

Les radicaux alkyle ou alcoxy inférieurs désignent de préférence des radicaux en $C_1$-$C_4$.

Les sels de ces composés particulièrement préférés sont les chlorhydrates, bromhydrates, sulfates ou tartrates.

Les paraphénylènediamines monosubstituées sur l'un des groupements amino, répondant à la formule (II) préférées, sont choisies parmi les composés répondant à la formule (II), dans laquelle $R_{12}$ désigne hydrogène ou méthyle, $R_{13}$ et/ou $R_{14}$ désigne(nt) hydrogène, méthyle, méthoxy ou un atome de chlore.

Parmi ces composés, ceux particulièrement préférés sont choisis parmi la N-β-méthoxyéthylparaphénylènediamine, la N-β-méthoxyéthyl 2-méthoxyparaphénylènediamine, la N-β-hydroxyéthyl 2-méthoxyparaphénylènediamine, la N-β-hydroxyéthylparaphénylènediamine, la N-β-méthoxyéthyl 2-chloroparaphénylènediamine, la N-β-hydroxyéthyl 2-chloroparaphénylènediamine, la N-β-hydroxyéthyl 2-méthylparaphénylènediamine et leurs sels, tels que les chlorhydrates, dichlorhydrates ou sulfates.

Le colorant indolique est utilisé de préférence dans la composition conforme à l'invention, dans des proportions comprises entre 0,1 et 5% en poids, et plus particulièrement entre 0,2 et 2% en poids, par rapport au poids total de la composition.

Les paraphénylènediamines monosubstituées sur l'un des groupes amino sont utilisées de préférence dans les compositions conformes à l'invention, dans des proportions comprises entre 0,05 et 1% en poids, et en particulier entre 0,1 et 0,5% en poids par rapport au poids total de la composition.

Les compositions conformes à l'invention contiennent dans une forme de réalisation préférée, d'autres colorants et notamment des colorants d'oxydation dits "rapides", c'est-à-dire des précurseurs de colorants à structure benzénique, susceptibles de générer les composés colorés par simple oxydation à l'air pendant le temps de pose sur la chevelure, généralement inférieur à 1 heure, et en l'absence de tout autre agent oxydant, tel que des ions iodure.

Ces colorants d'oxydation "rapides" sont plus particulièrement choisis parmi les dérivés trihydroxylés du benzène, tels que le 1,2,4-trihydroxybenzène et le 2,4,5-trihydroxytoluène ou leurs formes protégées, telles que le triacétate.

Conformément à l'invention, ces colorants d'oxydation rapides sont utilisés dans des proportions comprises entre 0,02 et 1% et de préférence entre 0,05 et 0,3% en poids par rapport au poids total de la composition.

Une forme de réalisation particulièrement préférée de l'invention est constituée par une composition contenant dans un milieu cosmétiquement acceptable, approprié pour la teinture, le colorant indolique et plus particulièrement le 5,6-dihydroxyindole, une paraphénylènediamine monosubstituée sur l'un des groupements amino et plus particulièrement les paraphénylènediamines préférées indiquées ci-dessus ou l'un de leurs sels et le 1,2,4-trihydroxybenzène ou son triacétate, dans des proportions suffisants pour teindre les cheveux et de préférence celles indiquées ci-dessus.

Le milieu aqueux approprié pour la teinture a un pH pouvant varier entre 4 et 11, et de préférence entre 6 et 9. Il est ajusté à la valeur désirée à l'aide d'agents alcalinisants ou acidifiants connus en eux-mêmes, notamment ceux utilisés en cosmétique lorsque les compositions sont destinées à être utilisées pour la teinture des cheveux humains.

Le milieu aqueux peut renfermer en outre des solvants qui doivent être cosmétiquement acceptables lorsque les compositions sont utilisées pour la teinture des cheveux humains.

Ces solvants sont utilisés dans des proportions pouvant aller jusqu'à 30% par rapport au poids total de la composition et sont choisis plus particulièrement parmi les alcools inférieurs, tels que l'alcool éthylique, l'alcool propylique ou isopropylique, l'alcool tertiobutylique; les glycols, tels que l'éthylèneglycol, le propylèneglycol; des éthers de glycol, tels que les éthers monométhylique, monoéthylique et monobutylique de l'éthylèneglycol, l'acétate du monoéthyléther de l'éthylèneglycol, le monométhyléther du propylèneglycol, du dipropylène glycol, le lactate de méthyle.

Les solvants préférés sont constitués par l'alcool éthylique, le propylèneglycol et l'éther monobutylique de l'éthylèneglycol.

Ces compositions peuvent adopter la forme de lotion épaissie ou non, de gel ou de mousse aérosol et renfermer des adjuvants bien connus en coloration capillaire pour ce type d'application, tels que des agents plastifiants, des agents antioxydants, des agents épaississants, des agents de conditionnement tels que des polymères cationiques, des agents tensio-actifs cationiques; elles peuvent également renfermer des agents tensio-actifs anioniques, non ioniques ou amphotères ainsi que des silicones, notamment des silicones volatiles.

Les agents épaississants sont choisis plus particulièrement parmi l'alginate de sodium, la gomme arabique, la gomme de guar ou de caroube, les hétérobiopolysaccharides tels que la gomme de xanthane, les dérivés de la cellulose tels que la méthylcellulose, l'hydroxyéthylcellulose, l'hydroxyméthylcellulose, l'hydroxypropylcellulose, la carboxyméthylcellulose et des polymères divers ayant des fonctions épaississantes tels que les dérivés d'acide acrylique.

On peut également utiliser des agents épaississants minéraux, tels que la bentonite. Ces agents épaississants sont présents de préférence dans des proportions comprises entre 0,1 et 5% en poids et en particulier entre 0,5 et 3% en poids par rapport au poids total de la composition.

Les compositions conformes à l'invention contiennent de préférence des agents anti-oxydants dans des proportions suffisantes pour éviter une oxydation prématurée des colorants et qui sont comprises de préférence entre 0,03 et 1% en poids. Ils sont choisis en particulier parmi les sulfites, hydrosulfites, et l'acide ascorbique.

Une forme de réalisation de l'invention peut également consister à stocker les composants de la composition dans des compartiments séparés, éventuellement à l'abri de l'air et à procéder au mélange des différents constituants au moment de leur emploi.

Le procédé de coloration des fibres kératiniques et en particulier des cheveux humains consiste à appliquer sur les fibres au moins une composition telle que définie ci-dessus pendant le temps suffisant pour développer à l'air une coloration des fibres kératiniques et en particulier des cheveux humains. Cette coloration s'effectue sans adjonction d'un catalyseur d'oxydation ou d'un agent oxydant autre que l'air.

Selon une forme de réalisation préférée, on applique la composition plusieurs fois de façon successive et on constate que la teinte obtenue devient de plus en plus foncée, ce que l'on appelle également la teinture progressive.

L'application de la composition est suivie après une durée de pose de 5 à 50 minutes par un rinçage, un shampooing éventuel, un nouveau rinçage et un séchage.

Les exemples suivants sont destinés à illustrer l'invention sans pour autant présenter un caractère limitatif.

EXEMPLE 1

On prépare la composition suivante :

| | | |
|---|---|---|
| - 5,6-dihydroxyindole | | 0,21 g |
| - Dichlorhydrate de N-ß-méthoxyéthyl paraphénylènediamine | | 0,33 g |
| - Gomme de xanthane vendue sous la dénomination RHODOPOL SC par la Société RHONE POULENC | | 2,0 g |
| - Alkyléther de glycoside vendu à la concentration de 60% MA sous la dénomination TRITON CG 110 par la Société SEPPIC | | 3,5 g MA |
| - Triéthanolamine | | 4,0 g |
| - Acide tartrique | | 0,3 g |
| - Alcool éthylique | | 10,0 g |
| - Conservateurs qs | | |
| - Eau | qsp | 100,0 g |

On réalise une application de cette composition sur des cheveux naturels gris à 90% de blancs.

On laisse poser 10 minutes. On rince et on sèche. On obtient des cheveux colorés dans une nuance blond foncé, alors qu'une application réalisée dans les mêmes conditions avec la compositions ne renfermant que le 5,6-dihydroxyindole conduit à un blond doré ou celle ne renfermant que le dichlorhydrate de N-β-méthoxyéthyl paraphénylènediamine ne teint pas.

Après trois applications de 10 minutes chacune, rinçage et séchage entre chaque application, on obtient avec ladite composition une nuance châtain très foncé mat, tandis qu'avec la même composition ne renfermant que le 5,6-dihydroxyindole, on obtient un châtain cendré et que le dichlorhydrate de N-β-méthoxyéthylparaphénylènediamine ne teint pas.

EXEMPLE 2

On prépare la composition suivante :

| | | | |
|---|---|---|---|
| - 5,6-dihydroxyindole | | 0,21 | g |
| - Dichlorhydrate de N-ß-méthoxyéthyl paraphénylènediamine | | 0,33 | g |
| - 1,2,4-trihydroxybenzène | | 0,18 | g |
| - Gomme de xanthane vendue sous la dénomination RHODOPOL SC par la Société RHONE POULENC | | 2,0 | g |
| - Alkyléther de glycoside vendu à la concentration de 60% MA sous la dénomination TRITON CG 110 par la Société SEPPIC | | 3,5 | g MA |
| - Triéthanolamine | | 4,0 | g |
| - Acide tartrique | | 0,3 | g |
| - Alcool éthylique | | 10,0 | g |
| - Conservateurs qs | | | |
| - Eau | qsp | 100,0 | g |

On réalise trois applications de cette composition sur des cheveux naturels gris à 90% de blancs. Chaque application correspond à un temps de pose de 10 minutes suivi d'un rinçage et d'un séchage. On obtient en final une nuance châtain très foncé à reflets chauds.

EXEMPLE 3

On prépare la composition suivante :

| | | | |
|---|---|---|---|
| - 5,6-dihydroxyindole | | 0,21 | g |
| - Sulfate de N-ß-méthoxyéthyl 2-méthoxy p-phénylènediamine | | 0,41 | g |
| - Gomme de xanthane vendue sous la dénomination RHODOPOL SC par la Société RHONE POULENC | | 2,0 | g |
| - Alkyléther de glycoside vendu à la concentration de 60% MA sous la dénomination TRITON CG 110 par la Société SEPPIC | | 3,5 | g MA |
| - Triéthanolamine | | 4,0 | g |
| - Acide tartrique | | 0,3 | g |
| - Alcool éthylique | | 10,0 | g |
| - Conservateurs qs | | | |
| - Eau | qsp | 100,0 | g |

On effectue trois applications de cette composition sur des cheveux naturels gris à 90% de blancs, de 10

minutes chacune, suivies chacune d'un rinçage et d'un séchage. On obtient en final une nuance gris moyen cendré, tandis que le 5,6-dihydroxyindole seul dans le même support conduit à un châtain cendré et que le sulfate de N-β-méthoxyéthyl 2-méthoxy-p-phénylène diamine seul donne un blond clair d'une nuance terne.

EXEMPLE 4

On reproduit l'exemple 3 par addition à la composition de 0,18 g de 1,2,4-trihydroxybenzène. Après trois applications, on obtient un gris moyen lumineux.

EXEMPLE 5

On prépare la composition suivante :

| | | | |
|---|---|---|---|
| - 5,6-dihydroxyindole | | 0,21 | g |
| - Sulfate de N-ß-hydroxyéthyl 2-méthoxy-p-phénylènediamine | | 0,39 | g |
| - Gomme de xanthane vendue sous la dénomination RHODOPOL SC par la Société RHONE POULENC | | 2,0 | g |
| - Alkyléther de glycoside vendu à la concentration de 60% MA sous la dénomination TRITON CG 110 par la Société SEPPIC | | 3,5 | g MA |
| - Triéthanolamine | | 4,0 | g |
| - Acide tartrique | | 0,3 | g |
| - Alcool éthylique | | 10,0 | g |
| - Conservateurs qs | | | |
| - Eau | qsp | 100,0 | g |

On effectue trois applications de cette composition sur des cheveux naturels gris à 90% de blancs. Chaque application correspond à un temps de pose de 10 minutes suivi d'un rinçage et d'un séchage. En final, la nuance est gris foncé cendré tandis que le 5,6-dihydroxyindole seul dans la même support donne un châtain cendré et que le sulfate de N-β-hydroxyéthyl 2-méthoxy p-phénylènediamine conduit à un blond clair irisé.

EXEMPLE 6

On prépare la composition suivante :

| | | | |
|---|---|---|---|
| - 5,6-dihydroxyindole | | 0,21 g | |
| - Sulfate de N-ß-hydroxyéthyl 2-méthylparaphénylènediamine | | 0,37 g | |
| - Gomme de xanthane vendue sous la dénomination RHODOPOL SC par la Société RHONE POULENC | | 2,0 g | |
| - Alkyléther de glycoside vendu à la concentration de 60% MA sous la dénomination TRITON CG 110 par la Société SEPPIC | | 3,5 g | MA |
| - Triéthanolamine | | 4,0 g | |
| - Acide tartrique | | 0,3 g | |
| - Alcool éthylique | | 10,0 g | |
| - Conservateurs qs | | | |
| - Eau | qsp | 100,0 g | |

On effectue trois applications de cette composition sur des cheveux naturels gris à 90% de blancs. Chaque application correspond à un temps de pose de 10 minutes suivi d'un rinçage et d'un séchage. En final, la nuance est gris foncé cendré tandis que le 5,6-dihydroxyindole seul dans le même support donne un châtain cendré et que le sulfate de N-β-hydroxyéthyl 2-méthyl p-phénylènediamine donne un blond clair très légèrement doré.

EXEMPLE 7

On prépare la composition suivante :

| | | | |
|---|---|---|---|
| - 5,6-dihydroxyindole | | 0,21 g | |
| - Sulfate de N-ß-hydroxyéthyl p-phénylènediamine | | 0,35 g | |
| - Gomme de xanthane vendue sous la dénomination RHODOPOL SC par la Société RHONE POULENC | | 2,0 g | |
| - Alkyléther de glycoside vendu à la concentration de 60% MA sous la dénomination TRITON CG 110 par la Société SEPPIC | | 3,5 g | MA |
| - Triéthanolamine | | 4,0 g | |
| - Acide tartrique | | 0,3 g | |
| - Alcool éthylique | | 10,0 g | |
| - Conservateurs qs | | | |
| - Eau | qsp | 100,0 g | |

On effectue trois applications de cette composition sur des cheveux naturels gris à 90% de blancs. Chaque

application correspond à un temps de pose de 10 minutes suivi d'un rinçage et d'un séchage. En final, la nuance est châtain très foncé à reflets violine, tandis que le 5,6-dihydroxyindole seul dans le même support donne un châtain cendré et que le sulfate de N-β-hydroxyéthyl-p-phénylènediamine donne un blond clair doré.

EXEMPLE 8

On prépare la composition suivante :

| | | |
|---|---|---|
| - 5,6-dihydroxyindole | | 0,21 g |
| - Chlorhydrate de N-ß-méthoxyéthyl 2-chloro-p-phénylènediamine | | 0,33 g |
| - Gomme de xanthane vendue sous la dénomination RHODOPOL SC par la Société RHONE POULENC | | 2,0 g |
| - Alkyléther de glycoside vendu à la concentration de 60% MA sous la dénomination TRITON CG 110 par la Société SEPPIC | | 3,5 g MA |
| - Triéthanolamine | | 4,0 g |
| - Acide tartrique | | 0,3 g |
| - Alcool éthylique | | 10,0 g |
| - Conservateurs qs | | |
| - Eau | qsp | 100,0 g |

Après trois applications de cette composition de 10 minutes chacune, suivies d'un rinçage puis d'un séchage à chaque fois, on obtient sur cheveux permanentés gris à 90% de blancs, une nuance châtain violine, tandis que le 5,6-dihydroxyindole seul donne dans les mêmes conditions du châtain cendré et que le chlorhydrate de N-β-méthoxyéthyl 2-chloro-p-phénylène diamine seul ne donne aucune coloration.

EXEMPLE 9

On reproduit l'exemple 8 en additionnant à la composition 0,18 g de 1,2,4-tridhyroxybenzène. On obtient après trois applications une nuance châtain violine lumineux.

EXEMPLE 10

On prépare la composition suivante :

| | | | |
|---|---|---|---|
| - 5,6-dihydroxyindole | | 0,21 | g |
| - N-ß-hydroxyéthyl 2-chloro-p-phénylènediamine | | 0,26 | g |
| - 1,2,4-trihydroxybenzène | | 0,18 | g |
| - Gomme de xanthane vendue sous la dénomination RHODOPOL SC par la Société RHONE POULENC | | 2,0 | g |
| - Alkyléther de glycoside vendu à la concentration de 60% MA sous la dénomination TRITON CG 110 par la Société SEPPIC | | 3,5 | g MA |
| - Triéthanolamine | | 4,0 | g |
| - Acide tartrique | | 0,3 | g |
| - Alcool éthylique | | 10,0 | g |
| - Conservateurs qs | | | |
| - Eau | qsp | 100,0 | g |

On réalise trois applications de cette composition sur des cheveux naturels gris à 90% de blancs. Chaque application correspond à un temps de pose de 10 minutes, suivi d'un rinçage et d'un séchage. On obtient en final une nuance châtain violine, tandis que l'association de 5,6-dihydroxyindole et de 1,2,4-trihydroxybenzène dans le même support donne un blond foncé cendré et que la N-β-hydroxyéthyl 2-chloro-p-phénylènediamine seule ne colore pas.

EXEMPLE 11

On prépare la composition suivante :

| | | | |
|---|---|---|---|
| - Monobromhydrate de 2-méthyl 5,6-dihydroxyindole | | 0,34 | g |
| - Sulfate de N-ß-hydroxyéthyl 2-méthylparaphénylènediamine | | 0,37 | g |
| - Alcool éthylique | | 10,0 | g |
| - Gomme de xanthane vendue sous la dénomination de RHODOPOL SC par la Société RHONE POULENC | | 2,0 | g |
| - Alkyléther de glycoside vendu sous la dénomination de TRITON CG 110 par la Société SEPPIC | | 2,1 | g MA |
| - Acide tartrique | | 0,3 | g |
| - Triéthanolamine | | 4,0 | g |
| - Conservateurs qs | | | |
| - Eau déminéralisée | qsp | 100,0 | g |

Ce gel est appliqué 10 minutes sur des cheveux gris permanentés à 90% de blancs. On rince et on sèche. Après trois applications, on obtient sur cheveux permanentés une nuance châtain clair nacré. Dans les mêmes conditions, le dérivé indolique ne donne qu'une coloration blond foncé cendré. Le dérivé de paraphénylènediamine, quant à lui, teint dans une nuance blond clair très légèrement doré.

EXEMPLE 12

On prépare la composition suivante :

| | | | |
|---|---|---|---|
| - 5-méthoxy 6-hydroxyindole | | 0,23 | g |
| - N-ß-hydroxyéthyl 2-chloroparaphénylènediamine | | 0,26 | g |
| - 1,2,4-trihydroxybenzène | | 0,18 | g |
| - Alcool éthylique | | 10,0 | g |
| - Gomme de xanthane vendue sous la dénomination de RHODOPOL SC par la Société RHONE POULENC | | 2,0 | g |
| - Alkyléther de glycoside vendu sous la dénomination de TRITON CG 110 par la Société SEPPIC | | 2,1 | g MA |
| - Acide tartrique | | 0,3 | g |
| - Triéthanolamine | | 4,0 | g |
| - Conservateurs qs | | | |
| - Eau déminéralisée | qsp | 100,0 | g |

On applique ce gel 10 minutes sur des cheveux gris permamentés à 90% de blancs. On rince et on sèche.

Après trois applications, on obtient une nuance blond foncé doré nacré. Dans ces conditions, le dérivé indolique et le dérivé de paraphénylènediamine ne teignent pratiquement pas les cheveux. Le trihydroxybenzène teint, quant à lui, dans une nuance blond clair beige nacré.

EXEMPLE 13

On prépare la composition suivante :

| | | | |
|---|---|---|---|
| - 2-carboxy 5,6-dihydroxyindole | | 0,5 | g |
| - Sulfate de N-β-hydroxyéthyl-p-phénylènediamine | | 0,8 | g |
| - Alcool éthylique | | 10,0 | g |
| - Lauryléthersulfate de sodium en solution aqueuse à 28% de MA | | 0,2 | g MA |
| - Hydroxyéthylcellulose vendue sous la dénomination NATROSOL 250 HHR par la Société AQUALON | | 1,0 | g |
| - Alkylétherglycoside vendu à 60% de MA sous la dénomination TRITON CG 110 par la Société ROHM & HAAS | | 5,0 | g MA |
| - Triéthanolamine qs pH=8 | | | |
| - Eau déminéralisée | qsp | 100,0 | g |

On réalise une application de cette composition sur des cheveux naturels gris à 90% de blancs.

On laisse poser 10 minutes. On rince et on sèche. On obtient des cheveux colorés dans une nuance cendré doré irisé, alors qu'une application réalisée dans les mêmes conditions avec la composition ne renfermant que le 2-carboxy 5,6-dihydroxyindole ne teint pas ou celle ne renfermant que le sulfate de N-β-hydroxy éthyl-p-phénylènediamine conduit à une nuance doré.

Après trois applications de 10 minutes chacune, rinçage et séchage entre chaque application, on obtient avec ladite composition une nuance cendré irisé intense, tandis qu'avec la même composition ne renfermant que le sulfate de N-β-hydroxyéthyl-p-phénylènediamine, on obtient une nuance doré et que le 2-carboxy, 5,6-dihydroxyindole ne teint pas.

EXEMPLE 14

On prépare la composition suivante :

| | | | |
|---|---|---|---|
| - 3-méthyl 5,6-dihydroxyindole | | 0,8 | g |
| - Sulfate de N-ß-hydroxyéthyl 2-chloroparaphénylènediamine | | 1,0 | g |
| - Alcool éthylique | | 10,0 | g |
| - Lauryléthersulfate de sodium en solution aqueuse à 28% de MA | | 0,2 | g MA |
| - Hydroxyéthylcellulose vendue sous la dénomination NATROSOL 250 HHR par la Société AQUALON | | 1,0 | g |
| - Alkylétherglycoside vendu à 60% de MA sous la dénomination TRITON CG 110 par la Société ROHM & HAAS | | 5,0 | g MA |
| - Triéthanolamine qs pH=6 | | | |
| - Eau déminéralisée | qsp | 100,0 | g |

On réalise trois applications de cette composition sur des cheveux permanentés gris à 90% de blancs. Chaque application correspond à un temps de pose de 10 minutes suivi d'un rinçage et d'un séchage. On obtient en final une nuance cendré mat profond.

EXEMPLE 15

On prépare la composition suivante :

| | | | |
|---|---|---|---|
| - 2,3-diméthyl 5,6-dihydroxyindole | | 1,5 | g |
| - Sulfate de N-ß-méthoxyéthyl-p-phénylènediamine | | 0,1 | g |
| - Alcool éthylique | | 10,0 | g |
| - Lauryléthersulfate de sodium en solution aqueuse à 28% de MA | | 0,2 | g MA |
| - Hydroxyéthylcellulose vendue sous la dénomination NATROSOL 250 HHR par la Société AQUALON | | 1,0 | g |
| - Alkylétherglycoside vendu à 60% de MA sous la dénomination TRITON CG 110 par la Société ROHM & HAAS | | 5,0 | g MA |
| - Triéthanolamine qs pH=7 | | | |
| - Eau déminéralisée | qsp | 100,0 | g |

On réalise trois applications de cette composition sur des cheveux naturels gris à 90% de blancs. Chaque application correspond à un temps de pose de 10 minutes suivi d'un rinçage et d'un séchage. On obtient en final une nuance blond foncé cendré irisé.

EXEMPLE 16

On prépare la composition suivante :

| | | | |
|---|---|---|---|
| - 2,3-diméthyl 5,6-dihydroxyindole | | 1,5 | g |
| - Sulfate de N-β-méthoxyéthyl-p-phénylènediamine | | 0,1 | g |
| - Alcool éthylique | | 30,0 | g |
| - Alkylétherglycoside vendu à 60% de MA sous la dénomination TRITON CG 110 par la Société ROHM & HAAS | | 2,4 | g MA |
| - Hydroxyde de sodium qs pH=9 | | | |
| - Eau déminéralisée | qsp | 100,0 | g |

On réalise trois applications de cette composition sur des cheveux permanentés gris à 90% de blancs. Chaque application correspond à un temps de pose de 10 minutes suivi d'un rinçage et d'un séchage. On obtient en final une nuance gris bleu profond.

EXEMPLE 17

On reproduit l'exemple 16 en additionnant à la composition 0,5 g de 2,4,5-trihydroxytoluène. On obtient après trois applications une nuance cendré violine tandis que la même composition ne renfermant pas le sulfate de N-β-méthoxyéthyl-p-phénylènediamine conduit à une nuance rose profond et que la composition ne renfermant pas le 2,3-diméthyl 5,6-dihydroxyindole conduit à une nuance blond foncé nacré.

EXEMPLE DE PREPARATION 1

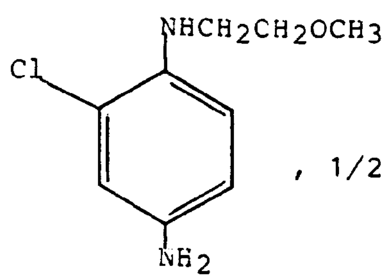

, 1/2 $H_2SO_4$

Préparation de l'hémisulfate hémihydrate de N(β-méthoxyéthyl)2-chloroparaphénylènediamine

1) Préparation du (β-méthoxyéthyl)amino-4 chloro-3 nitrobenzène

On chauffe au reflux pendant 3 heures 0,3 mole (56,2 g) de dichloro-3,4 nitrobenzène dans 170 ml de méthoxy-2 éthylamine.

Le produit attendu est précipité par dilution dans 500 ml d'eau glacée. Après réempatage dans l'eau puis séchage, le produit obtenue est recristallisé de l'acétone. Il fond à 133°C.

L'analyse du produit obtenu donne les résultats suivants :

Analyse pour $C_9H_{11}ClN_2O_3$

| | C | H | N | Cl |
|---|---|---|---|---|
| Calculé | 46,86 | 4,81 | 12,15 | 15,37 |
| Trouvé | 46,81 | 4,99 | 12,17 | 15,17 |

2) Préparation de l'hémisulfate hémihydrate de N-(β-méthoxyéthyl)2-chloroparaphénylènediamine

A une suspension de 23 g de zinc en poudre dans 55 ml d'éthanol et 11 ml d'eau additionnée de 0,5 g de chlorure d'ammonium portée au reflux, on ajoute 10,6 g de (β-méthoxyéthyl)amino-4 chloro-3 nitrobenzène par portions. 5 minutes après la fin de l'addition, le zinc est éliminé par filtration à chaud du milieu réactionnel.

Le produit attendu précipite après ajout de 4,5 ml d'acide sulfurique au filtrat.

L'analyse du produit recristallisé du méthanol donne les résultats suivants :

Analyse pour $C_9H_{15}ClN_2O_{3,5}S_{0,5}$

| | C | H | N | S |
|---|---|---|---|---|
| Calculé | 41,78 | 5,84 | 10,82 | 6,20 |
| Trouvé | 41,57 | 5,81 | 10,95 | 6,59 |

Par dilution du filtrat par 4 ml d'acide chlorhydrique concentré, on prépare le chlorhydrate de N-(β-méthoxyéthyl)2-chloroparaphénylènediamine.

EXEMPLE DE PREPARATION 2

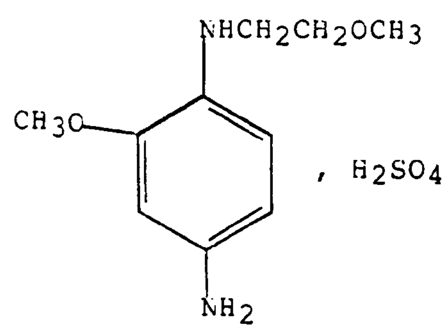

Préparation du sulfate de N-(β-méthoxyéthyl)2-méthoxyparaphénylènediamine

1) Préparation du (β-méthoxyéthyl)amino-4 méthoxy-3 nitrobenzène

On chauffe au reflux pendant 18 heures 0,3 mole (56,2 g) de chloro-4 méthoxy-3 nitrobenzène dans 170 ml de méthoxy-2 éthylamine. La méthoxy-2 éthylamine en excès est distillée. Le milieu réactionnel est dilué par 300 g d'eau glacée.

Le produit attendu précipite. Après recristallisation de l'éthanol puis du benzène, il fond à 113°C.

L'analyse du produit obtenu donne les résultats suivants :

Analyse pour $C_{10}H_{14}N_2O_4$

| | C | H | N |
|---|---|---|---|
| Calculé | 53,09 | 6,24 | 12,38 |
| Trouvé | 53,36 | 6,36 | 12,29 |

2) Préparation du sulfate de N-(β-méthoxyéthyl)2-méthoxy paraphénylènediamine

A une suspension de 60 g de zinc en poudre dans 120 ml d'éthanol et 18 ml d'eau additionnée de 1,2 g de chlorure d'ammonium portée au reflux, on ajoute 26,5 g de (β-méthoxyéthyl)amino-4 méthoxy-3 nitrobenzène. 5 minutes après la fin de l'addition, le zinc est éliminé par filtration. Par dilution du filtrat par 8 ml d'acide sulfurique, on précipite le produit attendu.

Après recristallisation de l'éthanol, les résultats de l'analyse sont :

Analyse pour $C_{10}H_{18}N_2SO_6$

| | C | H | N | S |
|---|---|---|---|---|
| Calculé | 40,81 | 6,17 | 9,52 | 10,87 |
| Trouvé | 41,00 | 6,10 | 9,38 | 10,98 |

## Revendications

**Revendications pour les Etats contractants suivants : AT, BE, CH, LI, DE, DK, FR, GB, GR, IT, NL, SE**

**1.** Composition tinctoriale pour fibres kératiniques, en particulier pour fibres kératiniques humaines, caractérisée par le fait qu'elle contient dans un milieu aqueux approprié pour la teinture :

a) un colorant indolique;

b) une paraphénylènediamine monosubstituée sur l'un des groupes amino, répondant à la formule :

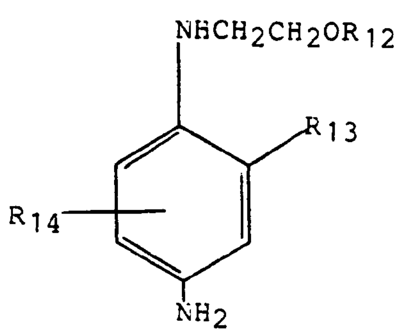

(II)

dans laquelle :

$R_{12}$ désigne un atome d'hydrogène ou un groupement alkyle inférieur;

$R_{13}$ désigne un atome d'hydrogène, un groupement alkyle inférieur, un groupement alcoxy inférieur ou un atome d'halogène;

$R_{14}$ désigne un atome d'hydrogène, un groupement alkyle inférieur, un groupement alcoxy inférieur ou un atome d'halogène; $R_{14}$ occupant l'une quelconque des positions restantes du noyau benzénique ainsi que les sels d'addition d'acide, cette composition ne contenant pas d'agent oxydant autre que l'air.

2. Composition selon la revendication 1, caractérisée par le fait que le colorant indolique répond à la formule :

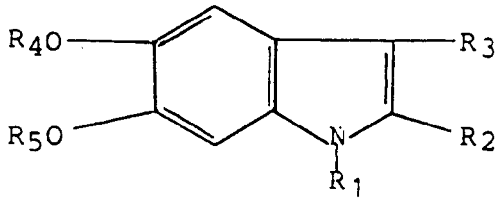

(I)

dans laquelle :

$R_1$ représente un atome d'hydrogène, un groupement alkyle inférieur ou un groupement $-SiR_6R_7R_8$;

$R_2$ et $R_3$, identiques ou différents, représentent un atome d'hydrogène, un groupement alkyle inférieur, un groupement carboxyle, un groupement alcoxy inférieur carbonyle ou un groupement $-COOSiR_6R_7R_6$;

$R_4$ et $R_5$, identiques ou différents, représentent un atome d'hydrogène, un groupement alkyle linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_6R_7R_6$, un groupement $-P(O)(OR_9)_2$, un groupement $SO_2OR_9$ ou bien $R_4$ et $R_5$, conjointement avec les atomes d'oxygène auxquels ils sont reliés, forment un cycle contenant éventuellement un groupement carbonyle, un groupement méthylène, un groupement thiocarbonyle ou un groupement :

$>P(O)OR_9$, ou bien $>CR_{10}R_{11}$;

$R_9$ et $R_{10}$ représentant un atome d'hydrogène ou un groupement alkyle inférieur, $R_{11}$ représente un groupement alcoxy inférieur ou un groupement mono- ou dialkylamino, $R_6$, $R_7$ et $R_8$, identiques ou différents, représentant des groupements alkyle inférieurs, linéaires ou ramifiés, et les sels d'addition avec des acides minéraux ou organiques ainsi que les sels de métaux alcalins, alcalino-terreux ou d'amines correspondants.

3. Composition selon la revendication 1, caractérisée par le fait que les composés de formule (I) sont choisis parmi le 5,6-dihydroxyindole, le 2-méthyl 5,6-dihydroxyindole, le 5-méthoxy 6-hydroxyindole, le (5 ou 6)-acétoxy(6- ou 5-)hydroxyindole, le 2-carboxy 5,6-dihydroxyindole, le 3-méthyl 5,6-dihydroxyindole, le 2,3-diméthyl 5,6-dihydroxyindole et leurs sels.

4. Composition selon l'une quelconque des revendications 1 à 3, caractérisé par le fait que les paraphénylènediamines monosubstituées sur l'un des groupements amino sont choisies parmi les composés de formule (II) dans laquelle $R_{12}$ désigne hydrogène ou méthyle, $R_{13}$ et $R_{14}$, indépendamment l'un de l'autre, désignent hydrogène, méthyle, méthoxy ou chlore.

5. Composition selon la revendication 4, caractérisée par le fait que les paraphénylènediamines monosubstituées sur l'un des groupements amino sont choisies parmi la N-β-méthoxyéthylparaphénylènediamine, la N-β-méthoxyéthyl 2-méthoxyparaphénylènediamine, la N-β-hydroxyéthylparaphénylènediamine, la N-β-hydroxyéthyl 2-méthylparaphénylènediamine, la N-β-méthoxyéthyl 2-chloroparaphénylènediamine, la N-β-hydroxyéthyl 2-chloroparaphénylènediamine et la N-β-hydroxyéthyl 2-méthoxyparaphénylènediamine.

6. Composition tinctoriale pour fibres kératiniques et en particulier pour fibres kératiniques humaines, caractérisée par le fait qu'elle contient dans un milieu cosmétiquement acceptable aqueux :

a) le 5,6-dihydroxyindole;

b) une paraphénylènediamine répondant à la formule (II), telle que définie dans la revendication 1, 4 ou 5.

7. Composition selon l'une quelconque des revendications 1 à 6, caractérisée par le fait que la paraphénylènediamine monosubstituée sur le groupement amino, répondant à la formule (II) est présente dans les compositions dans des proportions comprises entre 0,05 et 1% et de préférence entre 0,1 et 0,5% en poids par rapport au poids total de la composition.

8. Composition selon l'une quelconque des revendications 1 à 7, caractérisée par le fait que le colorant indolique est présent dans la composition dans des proportions comprises entre 0,1 et 5% en poids et de préférence entre 0,2 et 2% en poids par rapport au poids total de la composition.

9. Composition selon l'une quelconque des revendications 1 à 8, caractérisée par le fait qu'elle contient également un colorant d'oxydation rapide choisi parmi les dérivés trihydroxylés du benzène et leurs dérivés protégés.

**10.** Composition selon la revendication 9, caractérisée par le fait que le colorant d'oxydation "rapide" est choisi parmi le 1,2,4-trihydroxybenzène et le 2,4,5-trihydroxytoluène ou leur triacétate.

**11.** Composition, selon l'une quelconque des revendications 1 à 10, caractérisé par le fait que le colorant d'oxydation "rapide" est présent dans la composition dans des proportions comprises entre 0,02 et 1% en poids et de préférence entre 0,05 et 0,3% en poids par rapport au poids total de la composition.

**12.** Composition selon l'une quelconque des revendications 1 à 11, caractérisé par le fait qu'elle contient le 5,6-dihydroxyindole, une paraphénylène diamine monosubstituée sur l'un des groupements amino, répondant à la formule (II) ou l'un de ses sels et le 1,2,4-trihydroxybenzène ou son triacétate.

**13.** Composition selon l'une quelconque des revendications 1 à 12, caractérisée par le fait que le milieu est à un pH compris entre 4 et 11 et de préférence comprise entre 6 et 9.

**14.** Composition selon l'une quelconque des revendications 1 à 13, caractérisé par le fait que le milieu aqueux contient jusqu'à 30% de solvant(s) choisi(s) parmi les alcools, les glycols, les éthers de glycol, le lactate de méthyle et l'acétate du monoéthyl éther de l'éthylèneglycol.

**15.** Composition selon l'une quelconque des revendications 1 à 14, caractérisée par le fait que les compositions sont sous forme de lotion épaissie ou non, de gel ou de mousse aérosol.

**16.** Procédé de coloration des fibres kératiniques et en particulier des cheveux humains, caractérisé par le fait que l'on applique sur ces fibres au moins une composition telle que définie dans l'une quelconque des revendications 1 à 15, dans des quantités et pendant une durée suffisante pour développer à l'air une coloration de ces fibres sans adjonction d'un agent oxydant.

**17.** Procédé selon la revendication 16, caractérisé par le fait que l'on procède à plusieurs applications successives de la composition telle que définie dans l'une quelconque des revendications 1 à 15, dans des quantités et pendant une durée suffisante pour obtenir une teinte de plus en plus foncée des fibres.

## Patentansprüche

### Patentansprüche für folgende Vertragsstaaten : AT, BE, CH, LI, DE, DK, FR, GB, GR, IT, NL, SE

**1.** Färbemittelzusammensetzung für Keratinfasern, insbesondere für menschliche Keratinfasern, dadurch **gekennzeichnet**, daß sie in einem für die Färbung geeignetem wäßrigen Milieu enthält:
a) einen Indolfarbstoff,
b) ein an einer der Aminogruppen monosubstituiertes Paraphenylendiamin gemäß der Formel

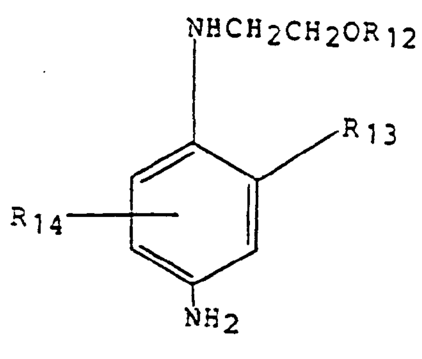

(II)

worin:

$R_{12}$ ein Wasserstoffatom oder eine Niederalkylgruppe bedeutet;

$R_{13}$ bedeutet ein Wasserstoffatom oder ein Niederalkylgruppe, eine Niederalkoxygruppe oder ein Halogenatom;

$R_{14}$ bedeutet eine Niederalkylgruppe, eine Niederalkoxygruppe oder ein Halogenatom; $R_{14}$ nimmt eine der freien Positionen des Benzolrings ein, sowie Säureadditionssalze davon, wobei diese Zusammensetzung kein anderes Oxidationsmittel als Luft enthält.

2. Zusammensetzung nach Anspruch 1, dadurch **gekennzeichnet**, daß der Indolfarbstoff der Formel entspricht

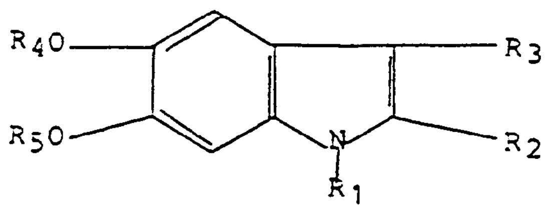

(I)

worin:

$R_1$ ein Wasserstoffatom, eine Niederalkylgruppe oder eine Gruppe $-SiR_6R_7R_8$ darstellt;

$R_2$ und $R_3$ sind gleich oder verschieden, und stellen ein Wassestoffatom, eine Niederalkylgruppe, eine Niedercarbonylalkoxygruppe oder eine $-COOSiR_6R_7R_8$ -Gruppe dar,

$R_4$ und $R_8$ sind gleich oder verschieden und stellen ein Wasserstoffatom, eine geradkettige oder verzweigte $C_1$-$C_{20}$-Alkylgruppe, eine Formylgruppe,eine geradkettige oder verzweigte $C_2$-$C_{20}$-Acylgruppe, eine geradkettige oder verzweigte $C_3$-$C_{20}$-Alkenoylgruppe, eine $-SiR_8R_7R_8$-Gruppe, eine $-P(O)(OR_9)_2$-Gruppe, eine $So_2OR_9$-Gruppe oder besser sind $R_4$ und $R_5$ zusammen mit den Sauerstoffatomen, an denen sie angeheftet sind, unter Bildung eines Rings verbunden, der gegebenenfalls eine Carbonylgruppe, eine Methylengruppe, eine Thiocarbonylgruppe oder eine Gruppe

$>P(O)OR_9$ oder besser eine $>CR_{10}R_{11}$- Gruppe enthält;

$R_9$ und $R_{10}$ entsprechen einem Wasserstoffatom oder einer Niederalkylgruppe, $R_{11}$ entspricht einer Niederalkoxygruppe oder einer Mono- oder Dialkylaminogruppe, $R_8$, $R_7$ und $R_8$ sind gleich oder verschieden und entsprechen geradkettigen oder verzweigten Niederalkylgruppen,

sowie ihre Additionssalze mit Mineralsäuren oder organischen Säuren, wie Alkalisalzen, Erdalkalisalzen oder entsprechenden Aminsalzen.

3. Zusammensetzung nach Anspruch 1, dadurch **gekennzeichnet**, daß die Verbindungen der Formel (I) ausgewählt sind aus 5,6-Dihydroxyindol, 2-Methyl-5,6-dihydroxyindol, 5-Methoxy-6-hydroxyindol, (5 oder 6)-Acetoxy(6- oder 5-)hydroxyindol, 2-Carboxy-5,6-dihydroxyindol, 3-Methyl-5,6-dihydroxyindol, 2,3-Dimethyl-5,6-dihydroxyindol und ihren Salzen.

4. Zusammensetzung nach einem der Ansprüche 1 bis 3, dadurch **gekennzeichnet**, daß sie an einer der Aminogruppen monosubstituierten Paraphenylendiamine ausgewählt sind aus Verbindungen der Formel (II), worin $R_{12}$ Wasserstoff oder Methyl, $R_{13}$ und $R_{14}$ unabhängig voneinander Wasserstoff, Methyl, Methoxy oder Chlor bedeuten.

5. Zusammensetzung nach Anspruch 4, dadurch **gekennzeichnet**, daß sie an einer der Aminogruppen monosubstituierten Paraphenylendiamine ausgewählt sind aus N-β-Methoxyethylparaphenylendiamin, N-β-Methoxyethyl-2-methoxyparaphenylendiamin, N-β-Hydroxyethylparaphenylendiamin, N-β-Hydroxyethyl-2-methylparaphenylendiamin, N-β-Hethoxyethyl-2-chlorparaphenylendiamin, N-β-Hydroxyethyl-2-chlorparaphenylendiamin und N-β-Hydroxyethyl-2-methoxyparaphenylendiamin.

6. Färbezusammensetzung für Keratinfasern und insbesondere für menschliche Keratinfasern, dadurch **gekennzeichnet**, daß sie in einem wäßrigen, kosmetisch annehmbaren Milieu enthält:

a) 5,6-Dihydroxyindol;

b) ein Paraphenylendiamin gemäß der Formel (II), wie Anspruch 1, 4 oder 5 definiert.

7. Zusammensetzung nach einem der Ansprüche 1 bis 6, dadurch **gekennzeichnet**, daß das an der Aminogruppe monosubstituierte Paraphenylendiamin gemäß der Formel (II) in der Zusammensetzung in einem Anteil zwischen 0,05 und 1 Gew.-%, und bevorzugt zwischen 0,1 und 0,5 Gew.-% bezogen auf das Gesamtgewicht der Zusammensetzung vorliegt.

8. Zusammensetzung nach einem der Ansprüche 1 bis 7, dadurch **gekennzeichnet**, daß der Indolfarbstoff

in der Zusammensetzung in einem Anteil zwischen 0,1 und 5 Gew.-% , und bevorzugt zwischen 0,2 und 2 Gew.-% bezogen auf das Gesamtgewicht der Zusammensetzung vorliegt.

9. Zusammensetzung nach einem der Ansprüche 1 bis 8, dadurch **gekennzeichnet**, daß sie auch einen "schnellen" Oxidationsfarbstoff enthält, ausgewählt aus Trihydroxyl-Derivaten des Benzols und seinen geschützten Derivaten.

10. Zusammensetzung nach Anspruch 9, dadurch **gekennzeichnet**, daß der "schnelle" Oxidationsfarbstoff ausgewählt ist aus 1,2,4-Trihydroxybenzol und 2,4,5-Trihydroxytoluol oder seinem Triacetat.

11. Zusammensetzung nach einem der Ansprüche 1 bis 10, dadurch **gekennzeichnet**, daß der "schnelle" Oxidationsfarbstoff in der Zusammensetzung in einem Anteil zwischen 0,02 und 1 Gew.-%, und bevorzugt zwischen 0,05 und 0,3 Gew.-% bezogen auf das Gesamtgewicht der Zusammensetzung vorliegt.

12. Zusammensetzung nach einem der Ansprüche 1 bis 11, dadurch **gekennzeichnet**, daß sie 5,6-Dihydroxyindol, ein an einer der Aminogruppen monosubstituiertes Paraphenylendiamin gemäß der Formel (II) oder ein Salz davon und 1,2,4-Trihydroxybenzol oder sein Triacetat enthält.

13. Zusammensetzung nach einem der Ansprüche 1 bis 12, dadurch **gekennzeichnet**, daß der pH des Milieus zwischen 4 und 11 ist, und bevorzugt zwischen 6 und 9 ist.

14. Zusammensetzung nach einem der Ansprüche 1 bis 13, dadurch **gekennzeichnet**, daß das wäßrige Milieu bis zu 30 % eines Lösungsmittels (von Lösungsmitteln), ausgewählt aus Alkoholen, Glykolen, Glykolethern, Methyllactat und Ethylenglykolmonoethyletheracetat enthält.

15. Zusammensetzung nach einem der Ansprüche 1 bis 14, dadurch **gekennzeichnet**, daß die Zusammensetzungen in Form einer viskosen oder nichtviskosen Lotion, Gel oder Schaumaerosol vorliegt.

16. Verfahren zur Färbung von Keratinfasern, insbesondere menschlichem Haar, dadurch **gekennzeichnet**, daß man auf die Fasern mindestens eine Zusammensetzung, wie in einem der Ansprüche 1 bis 15 definiert, in einer Menge und in einem Zeitraum, der zur Entwicklung einer Färbung der Fasern an der Luft ohne Zusatz eines Oxidationsmittels ausreicht, aufbringt.

17. Verfahren nach Anspruch 16, dadurch **gekennzeichnet**, daß man mehrere Anwendungen der Zusammensetzung hintereinander, wie in einem der Ansprüche 1 bis 15 definiert, in Mengen und für einen Zeitraum, der zum Erhalt einer immer dunkleren Färbung der Fasern ausreichend ist, durchführt.

## Claims

### Claims for the following Contracting States : AT, BE, CH, LI, DE, DK, FR, GB, GR, IT, NL, SE

1. Dyeing composition for keratinous fibres, especially for human fibres,characterized in that it contains, in an aqueous medium suitable for dyeing :
   a) an indole dyeing; and
   b) a para-phenylenediamine monosubstituted on one of the amino groups, corresponding to the formula :

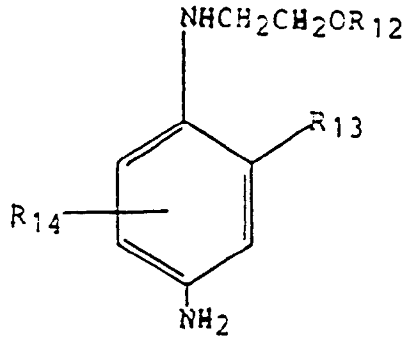

(II)

in which :

$R_{12}$ denotes a hydrogen atom or a lower alkyl group;

$R_{13}$ denotes a hydrogen atom, a lower alkyl group, a lower alkoxy group or a halogen atom; and

$R_{14}$ denotes a hydrogen atom, a lower alkyl group, a lower alkoxy group or a halogen atom; $R_{14}$ occupying any one of the remaining positions of the benzene ring; as well as the addition salts with an acid, this composition not containing an oxidizing agent other than air.

**2.** Composition according to Claim 1, characterized in that the indole dye corresponds to the formula :

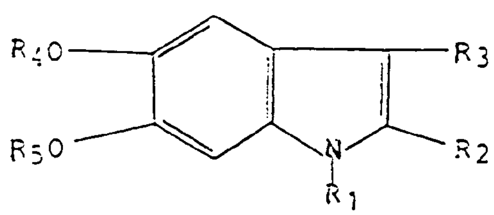

(I)

in which :

$R_1$ represents a hydrogen atom, a lower alkyl group or a group $-SiR_6R_7R_8$;

$R_2$ and $R_3$, which may be identical or different, represent a hydrogen atom, a lower alkyl group, a carboxyl group , a (lower alkoxy)carbonyl group or a group $-COOSiR_8R_7R_8$;

$R_4$ and $R_8$, which may be identical or different, represent a hydrogen atom, a linear or branched $C_1$-$C_{20}$ alkyl group, a formyl group, a linear or branched $C_2$-$C_{20}$ acyl group, a linear or branched $C_3$-$C_{20}$ alkenoyl group, a group $-SiR_8R_7R_8$, a group $-P(O)(OR_9)_2$ or a group $SO_2OR_9$, or alternatively $R_4$ and $R_5$, together with the oxygen atoms to which they are attached, form a ring optionally containing a carbonyl group, a methylene group, a thiocarbonyl group or a group :

$>P(O)OR_9$ or alternatively $>CR_{10}R_{11}$;

$R_9$ and $R_{10}$ representing a hydrogen atom or a lower alkyl group, and $R_{11}$ represents a lower alkoxy group or a mono- or dialkylamino group, $R_6$,$R_7$ and $R_8$, which may be identical or different, representing linear or branched lower alkyl groups; and the addition salts with inorganic or organic acids as well as the corresponding alkali metal , alkaline earth metal or amine salts.

**3.** Composition according to Claim 1, characterized in that the compounds of formula (I) are selected from 5,6-dihydroxyindole, 2-methyl-5,6-dihydroxyindole, 5-methoxy -6-dihydroxyindole, (5 or 6)-acetoxy-(6 or 5)-hydroxyindole, 2-carboxy-5,6-dihydroxyindole, 3-methyl-5,6-dihydroxyindole,2,3-dimethyl-5,6-dihydroxyindole and their salts.

**4.** Composition according to any one of Claims 1 to 3, characterized in that the para-phenylenediamines monosubstituted on one of the amino groups are selected from the compounds of the formula (II) in which $R_{12}$ denotes hydrogen or methyl and $R_{13}$ and $R_{14}$, independently of one another, denote hydrogen, methyl, methoxy or chlorine.

**5.** Composition according to Claim 4, characterized in that the para-phenylenediamines monosubstituted on one of the amino groups are selected from N-(β-methoxyethyl)-para-phenylenediamine, N-(β-methoxyethyl)-2-methoxy-para-phenylenediamine, N-(β-hydroxyethyl)-para-phenylenediamine, N-(β-hydroxyethyl)-2-methyl-para-phenylenediamine, N-(β-methoxyethyl)-2-chloro-para-phenylenediamine, N-(β-hydroxyethyl)-2-chloro-para-phenylenediamine and N-(β-hydroxyethyl)-2-methoxy-para-phenylenediamine.

**6.** Dyeing composition for keratinous fibres, and especially for human keratinous fibres, characterized in that it contains in a cosmetically acceptable aqueous medium :
a) 5,6-dihydroxyindole; and
b) a para-phenylenediamine corresponding to the formula (II) as defined in Claim 1, 4 or 5.

**7.** Composition according to any one of Claims 1 to 6, characterized in that the para-phenylenediamine monosubstituted on the amino group, corresponding to the formula (II), is present in the compositions in proportions of between 0.05 and 1%, and preferably between 0.1 and 0.5% by weight relative to the total weight of the composition.

**8.** Composition according to any one of Claims 1 to 7, characterized in that the indole dye is present in the composition in proportions of between 0.1 and 5% by weight, and preferably between 0.2 and 2% by weight , relative to the total weight of the composition.

**9.** Composition according to any one of Claims 1 to 8, characterized in that it also contains a rapid oxidation dye selected from trihydroxylated derivatives of benzene and their protected derivatives.

**10.** Composition according to Claim 9, chararacterized in that the "rapid" oxidation dye is selected from 1,2,4-trihydroxybenzene and 2,4,5-trihydroxytoluene or their triacetates.

**11.** Composition according to any one of Claims 1 to 10, characterized in that the "rapid" oxidation dye is present in the composition in proportions of between 0.02 and 1% by weight, and preferably between 0.05 and 0.3% by weight, relative to the total weight of the composition.

**12.** Composition according to any one of Claims 1 to 11, characterized in that it contains 5,6-dihydroxyindole ,a para-phenylenediamine monosubstituted on one of the amino groups, corresponding to the formula (II), or one of its salts, and 1,2,4-trihydroxybenzene or its triacetate.

**13.** Composition according to any one of Claims 1 to 12, characterized in that the medium is at a pH of between 4 and 11 and preferably between 6 and 9.

**14.** Composition according to any one of Claims 1 to 13, characterized in that the aqueous medium contains up to 30% of solvent(s) selected from alcohols, glycols, glycol ethers, methyl lactate and ethylene glycol monoethyl ether acetate.

**15.** Composition according to any one of Claims 1 to 14, characterized in that the compositions are in the form of a lotion, thickened or otherwise, a gel or an aerosol mousse.

**16.** Process for dyeing keratinous fibres, and especially human hair, characterized in that at least one composition as defined in any one of Claims 1 to 15 is applied on these fibres in amounts and for a sufficient time to develop in the air a coloration of these fibres without the addition of an oxidizing agent.

**17.** Process according to Claim 16, characterized in that several successive applications of the composition as defined in any one of Claims 1 to 15 are performed, in amounts and for a sufficient time to obtain and increasingly dark tinting of the fibres.